# EUROPEAN PATENT SPECIFICATION

(11) **EP 2 107 907 B1**
(45) Date of publication and mention of the grant of the patent: **01.02.2012**
(21) Application number: 07848600.8
(22) Date of filing: 18.12.2007
(51) Int. Cl.: A61K 31/519, A61P 37/00, A61P 9/00, A61K 31/403

(54) **Combination of pemirolast and ramatroban for use in the treatment of inflammatory disorders**
Kombination von Pemirolast und Ramatroban zur Verwendung bei der Behandlung von entzündlichen Erkrankungen
Combinaison de pémirolast et ramatrobane pour l'utilisation dans le traitement de troubles inflammatoires

(30) Priority: 20.12.2006 US 875850 P; 05.07.2007 US 929623 P
(43) Date of publication of application: 14.10.2009
(73) Proprietor: Cardoz AB, 103 12 Stockholm (SE)
(72) Inventor: RAUD, Johan, 10312 Stockholm (SE)
(74) Representative: McNeeney, Stephen Phillip
(86) International application number: PCT/GB2007/004863
(87) International publication number: WO 2008/075028

(56) References cited:
- EP-A1- 0 316 174
- EP-A1- 0 766 963
- EP-A1- 1 285 921
- US-A- 4 122 274
- US-A- 4 965 258
- US-B1- 6 362 214
- YANAGIHARA Y ET AL: "Immunopharmacological studies on TBX, a new antiallergic drug. (4) Effects on type II to IV allergic reactions and immunological functions in animal models" JAPANESE JOURNAL OF PHARMACOLOGY, THE JAPANESE PHARMACOLOGICAL SOCIETY, KYOTO, JP, vol. 51, no. 1, 1989, pages 93-100, XP008066536 ISSN: 0021-5198
- ISHIZUKA T: "THROMBOXANE A2 RECEPTOR BLOCKADE PREVENTS ATHEROSCLEROTIC PROCESS BY ITS ANTI-INFLAMMATORY EFFECT" VASCULAR DISEASE PREVENTION, BENTHAM SCIENCE PUBLISHERS, GB, vol. 3, no. 2, May 2006 (2006-05), pages 143-148, XP008078040 ISSN: 1567-2700
- ISHIZUKA T ET AL: "RAMATROBAN (BAY U 3405): A NOVEL DUAL ANTAGONIST OF TXA2 RECEPTOR AND CRTH2, A NEWLY IDENTIFIED PROSTAGLANDIN D2 RECEPTOR" CARDIOVASCULAR DRUG REVIEWS, NEVA PRESS, BRANFORD, CT, US, vol. 22, no. 2, June 2004 (2004-06), pages 71-90, XP008078031 ISSN: 0897-5957

## Description

### Field of the Invention

This invention relates to a novel pharmaceutical combination.

### Background and Prior Art

Cardiovascular diseases, such as coronary heart disease and stroke are major causes of death, disability, and healthcare expense, particularly in industrialised countries. Such diseases are often direct sequelae of atherosclerosis, a multifactorial condition that develops preferentially in subjects that smoke and/or present risk factors such as hypertension, diabetes mellitus, hypercholesterolemia, elevated plasma low density lipoprotein (LDL) and triglycerides.

Atherosclerotic lesions (or plaques) often develop over several years and sometimes decades. Pathological processes such as cholesterol accumulation in the artery wall, foam cell formation, inflammation and cell proliferation are typically involved.

Levels of high-density lipoproteins (HDLs), LDLs, total, cholesterol and triglycerides are all indicators is determining the risk of developing atherosclerosis and associated cardiovascular disorders, such as coronary artery diseases (e.g. angina pectoris, myocardial infarction, etc.), stroke (including cerebro-vascular accident and transient ischaemic attack) and peripheral arterial occlusive disease.

Patients with high overall cholesterol and/or triglycerides levels are at a significant risk, irrespective of whether or not they also have a favourable HDL level. Patients with normal overall cholesterol levels but low HDL levels are also at increased risk. Recently, it has also been noted that the level of risk of cardiovascular disease associated with high levels of apolipoprotein B (ApoB; which carries lipids in very low-density lipoproteins (VLDLs) and LDLs), and/or low levels of apolipoprotein A-I (ApoA-I; which carries lipids in HDLs), is extremely high.

Drugs that reduce LDL levels in serum can reduce the build-up of atherosclerotic plaques, and can reduce (long term) the risk of plaque rupture and associated thrombo-embolic complications. There are several types of drugs that can help reduce blood cholesterol levels. The most commonly prescribed are the hydroxymethylglutaryl-CoA (HMG-CoA) reductase inhibitors (hereinafter defined together, irrespective of their generic name, as "statins"), including simvastatin and atorvastatin. These drugs prevent directly the formation of cholesterol in the liver and thus reduce the risk of cardiovascular disease.

Other prescribed drug categories include resins (such as cholestyramine and colestipol), which act by binding bile acids, so causing the liver to produce more of the latter, and using up cholesterol in the process. Further, the B vitamin niacin has been reported at high doses to lower triglycerides and LDL levels in addition to increasing HDL levels. Fibrates (such as gemfibrozil and fenofibrate) are known to lower triglycerides and can increase HDL levels.

The introduction of cholesterol lowering drugs such as statins has significantly reduced mortality from coronary heart disease and stroke. However, these drugs suffer from the disadvantage that they are not equally effective in all patients and are known to have certain side effects (e.g. changes in liver function, myopathy and rhabdomyolysis), and atherosclerosis remains a major cause of death and disability. Indeed, a recent review article (Briel et al, JAMA, 295, 2046 (2006)) suggests that statins do not reduce serious cardiovascular events during the first four months of treatment in patients with acute coronary syndromes.

There is thus a real clinical need for safer and/or more effective treatments of atherosclerosis and associated cardiovascular disorders, particularly in those patients with acute coronary syndromes.

Pemirolast is an orally-active anti-allergic drug which is used in the treatment of conditions such as asthma, allergic rhinitis and conjunctivitis. See, for example, US patent No. 4,122,274, European Patent Applications EP 316 174 and EP 1 285 921, Yanagihara et al, Japanese Journal of Pharmacology, 51, 93 (1989) and Drugs of Today, 28, 29 (1992). The drug is presently marketed in e.g. Japan as the potassium salt.

Ramatroban is a thromboxane A2 antagonist and is known to have platelet aggregation inhibitory activity (see, for example, US patents Nos. 4,965,258 and 6,362,214). The drug is known to be of potential utility in the treatment of allergic and inflammatory diseases, such as asthma, as well as the treatment of prevention of thrombosis and thromboembolism (see, for example, Ishizuka et al, Cardiovascular Drug Reviews, 22, 71 (2004)). Ramatroban is presently marketed in e.g. Japan for the treatment of the treatment of allergic rhinitis (see, for example, Masuyama, Clin. Exp. All. Rev., 4, 27 (2004)).

Studies have been reported that relate to the potential use of pemirolast in the prevention of restenosis (Miyazawa et al, J. Cardiovasc. Pharmacol., 30, 157 (1997) and Ohsawa et al, Am. Heart J., 136, 1081 (1998) and J. Cardiol. 42, 13 (2003)). See also European patent application EP 766 693, which discloses that pemirolast exhibits an inhibitory effect on the proliferation of vascular smooth muscle cells.

Studies have also been reported that relate to the potential use of ramatroban in the prevention of restenosis (see Ishizuka et al, J. Cardiovasc. Pharmacol., 41, 571-8 (2003) and Vascular Disease Prevention, 3, 143 (2006)).

The use of combination products comprising, specifically, pemirolast and ramatroban is not disclosed in any of the above-mentioned documents. Further, the use of such combination products in the treatment of atherosclerosis and associated cardiovascular disorders, particularly in those patients with acute coronary syndromes, is not disclosed in any of these documents.

### Disclosure of the Invention

According to the invention, there is provided a combination product comprising:
(a) pemirolast, or a pharmaceutically-acceptable salt or solvate thereof; and
(b) ramatroban, or a pharmaceutically-acceptable salt or solvate thereof,
which combination products are referred to hereinafter as "the combination products according to the invention".

Pharmaceutically-acceptable salts that may be mentioned include acid addition salts and base addition salts. Such salts may be formed by conventional means, for example by reaction of a free acid or a free base form of an active ingredient with one or more equivalents of an appropriate acid or base, optionally in a solvent, or in a medium in which the salt is insoluble, followed by removal of said solvent, or said medium, using standard techniques (e.g. *in vacuo,* by freezedrying or by filtration). Salts may also be prepared by exchanging a counter-ion of an active ingredient in the form of a salt with another counter-ion, for example using a suitable ion exchange resin.

Preferred salts of pemirolast include pemirolast sodium and, more preferably, pemirolast potassium.

Active ingredients that are employed in combination products according to the invention (and in particular ramatroban) may be employed in diastereomericallyenriched and/or enantiomerically-enriched form. By "diastereomericallyenriched" and "enantiomerically-enriched" we mean, respectively, any mixture of the diastereoisomers/enantiomers of an active ingredient, in which one isomer is present in a greater proportion than the other. For example, enantiomers (of e.g. ramatroban) with optical purities (enantiomeric excess; e.e.) of greater than 90% may be employed. Preferred enantiomers of ramatroban include the R-enantiomer.

Combination products according to the invention provide for the administration of pemirolast as hereinbefore defined in conjunction with ramatroban as hereinbefore defined, and may thus be presented either as separate formulations, wherein at least one of those formulations comprises pemirolast, and at least one comprises ramatroban, or may be presented (i.e. formulated) as a combined preparation (i.e. presented as a single formulation including pemirolast and ramatroban).

Thus, there is further provided:
(1) a pharmaceutical formulation including pemirolast, or a pharmaceutically-acceptable salt or solvate thereof; ramatroban, or a pharmaceutically-acceptable salt or solvate thereof; and a pharmaceutically-acceptable adjuvant, diluent or carrier (which formulation is hereinafter referred to as a "combined preparation"); and
(2) a kit of parts comprising components:
   (A) a pharmaceutical formulation including pemirolast, or a pharmaceutically-acceptable salt or solvate thereof, in admixture with a pharmaceutically-acceptable adjuvant, diluent or carrier; and
   (B) a pharmaceutical formulation including ramatroban, or a pharmaceutically-acceptable salt or solvate thereof, in admixture with a pharmaceutically-acceptable adjuvant, diluent or carrier,
which components (A) and (B) are each provided in a form that is suitable for administration in conjunction with the other.

According to a further aspect of the invention, there is provided a method of making a kit of parts as defined above, which method comprises bringing component (A), as defined above, into association with a component (B), as defined above, thus rendering the two components suitable for administration in conjunction with each other.

By bringing the two components "into association with" each other, we include that components (A) and (B) of the kit of parts may be:
(i) provided as separate formulations (i.e. independently of one another), which are subsequently brought together for use in conjunction with each other in combination therapy; or
(ii) packaged and presented together as separate components of a "combination pack" for use in conjunction with each other in combination therapy.

Thus, there is further provided a kit of parts comprising:
(I) one of components (A) and (B) as defined herein; together with
(II) instructions to use that component in conjunction with the other of the two components.

The kits of parts described herein may comprise more than one formulation including an appropriate quantity/dose of pemirolast/salt/solvate, and/or more than one formulation including an appropriate quantity/dose of ramatroban/salt/solvate, in order to provide for repeat dosing. If more than one formulation (comprising either active compound) is present, such formulations may be the same, or may be different in terms of the dose of either compound, chemical composition(s) and/or physical form(s).

The combination products according to the invention find utility in the treatment of inflammatory conditions. Inflammatory conditions are typically characterized by activation of immune defence mechanisms, resulting in an effect that is more harmful than beneficial to the host. Such conditions are generally associated with varying degrees of tissue redness or hyperemia, swelling, hyperthermia, pain, itching, cell death and tissue destruction, cell proliferation, and/or loss of function. Inflammatory conditions that may be mentioned include cystitis, prostatitis, diabetic vascular complications, migraine and, more preferably, allergy (including allergic conjunctivitis and allergic rhinitis), ankylosing spondylitis, asthma, atopic dermatitis, chronic obstructive pulmonary disease, contact dermatitis, gouty arthritis, inflammatory bowel disease (such as Crohn's disease and ulcerative colitis), multiple sclerosis, osteoarthritis, pancreatitis, psoriasis, psoriatic arthritis, rheumatoid arthritis, tendinitis, bursitis, Sjogren's syndrome, systemic lupus erythematosus, uveitis, urticaria, vasculitis, atherosclerosis and associated cardiovascular disorders. Conditions that may be mentioned include migraine and, more preferably, asthma, chronic obstructive pulmonary disease, Crohn's disease, multiple sclerosis, psoriasis, rheumatoid arthritis, systemic lupus - erythematosus, ulcerative colitis and, more particularly, atherosclerosis and associated cardiovascular disorders.

The term "atherosclerosis" will be understood by those skilled in the art to include any disease characterised by cholesterol accumulation in a blood vessel, especially an artery wall, foam cell formation, inflammation and cell proliferation. Cardiovascular disorders "associated with" atherosclerosis include aortic aneurysms (including abdominal and/or atherosclerotic aortic aneurysms) and, more preferably, arteriosclerosis, peripheral arterial occlusive disease, coronary artery diseases (e.g. angina pectoris, myocardial infarction, heart attack, etc), coronary disease (including cardiac disease and heart disease, such as ischemic heart disease), and may also include plaque or atheroma rupture and/or instability, vascular or arterial disease, ischemic disease/ischemia and stroke (including cerebro-vascular accident and transient ischaemic attack).

Patient groups that may be mentioned include those with acute coronary syndromes. The term "acute coronary syndrome(s)" will be understood by the skilled person to include any abnormal myocardial and ischemic state, often but not exclusively associated with, for example the development of, chest pain (e.g. of a cardiac nature) and/or an abnormal electrocardiogram (ECG). Such syndromes are the most common presentation of myocardial infarction (heart attack). The skilled person will appreciate that the term is largely synonymous with the term "unstable angina", as opposed to "stable angina" (i.e. angina that develops during exertion and resolves at rest). Exertional angina that occurs at worsening rate ("crescendo angina") will similarly be regarded by the skilled person as within the definition "unstable".

According to a further aspect of the invention there is provided a use of a combination product according to the invention in a method of treatment of an inflammatory disorder, and in particular atherosclerosis and/or an associated cardiovascular disorder, which method comprises the administration of such a combination product to a patient in need of such treatment.

For the avoidance of doubt, in the context of the present invention, the terms "treatment", "therapy" and "therapy method" include the therapeutic, or palliative, treatment of patients in need of, as well as the prophylactic treatment and/or diagnosis of patients which are susceptible to, inflammatory disorders, such as atherosclerosis and associated cardiovascular disorders.

With respect to the kits of parts as described herein, by "administration in conjunction with", we include that respective formulations comprising pemirolast (or salt/solvate thereof) and ramatroban (or salt/solvate thereof) are administered, sequentially, separately and/or simultaneously, over the course of treatment of the relevant condition.

Thus, in respect of the combination product according to the invention, the term "administration in conjunction with" includes that the two components of the combination product (pemirolast and ramatroban) are administered (optionally repeatedly), either together, or sufficiently closely in time, to enable a beneficial effect for the patient, that is greater, over the course of the treatment of the relevant condition, than if either a formulation comprising pemirolast, or a formulation comprising ramatroban, are administered (optionally repeatedly) alone, in the absence of the other component, over the same course of treatment. Determination of whether a combination provides a greater beneficial effect in respect of, and over the course of treatment of, a particular condition will depend upon the condition to be treated or prevented, but may be achieved routinely by the skilled person.

Further, in the context of a kit of parts according to the invention, the term "in conjunction with" includes that one or other of the two formulations may be administered (optionally repeatedly) prior to, after, and/or at the same time as, administration of the other component. When used in this context, the terms "administered simultaneously" and "administered at the same time as" include that individual doses of pemirolast and ramatroban are administered within 48 hours (e.g. 24 hours) of each other.

"Patients" include mammalian (including human) patients.

In accordance with the invention, pemirolast and ramatroban are preferably administered locally or systemically, for example orally, intravenously or intraarterially (including by intravascular stent and other perivascular devices/dosage forms), intramuscularly, cutaneously, subcutaneously, transmucosally (e.g. sublingually or buccally), rectally, transdermally, nasally, pulmonarily (e.g. tracheally or bronchially), topically, or any other parenteral route, in the form of a pharmaceutical preparation comprising the compound(s) in pharmaceutically acceptable dosage form(s). Preferred modes of delivery include oral (particularly), intravenous, cutaneous or subcutaneous, nasal, intramuscular, or intraperitoneal delivery.

Pemirolast and ramatroban will generally be administered together or separately in the form of one or more pharmaceutical formulations in admixture with a pharmaceutically acceptable adjuvant, diluent or carrier, which may be selected with due regard to the intended route of administration and standard pharmaceutical practice. Such pharmaceutically acceptable carriers may be chemically inert to the active compounds and may have no detrimental side effects or toxicity under the conditions of use. Such pharmaceutically acceptable carriers may also impart an immediate, or a modified, release of either active ingredient, whether administered together in a combined preparation or in the form of a kit of parts.

Suitable pharmaceutical formulations may be commercially available or otherwise are described in the literature, for example, Remington The Science and Practice of Pharmacy, 19th ed., Mack Printing Company, Easton, Pennsylvania (1995) and Martindale - The Complete Drug Reference (34th Edition) and the documents referred to therein . Otherwise, the preparation of suitable formulations, and in particular combined preparations including both pemirolast and ramatroban may be achieved non-inventively by the skilled person using routine techniques.

The amount of active ingredients in the formulation(s) will depend on the severity of the condition, and on the patient, to be treated, as well as the compound(s) which is/are employed, but may be determined non-inventively by the skilled person.

Depending on the disorder, and the patient, to be treated, as well as the route of administration, active ingredients may be administered at varying therapeutically effective doses to a patient in need thereof.

However, the dose administered to a mammal, particularly a human, in the context of the present invention should be sufficient to effect a therapeutic response in the mammal over a reasonable timeframe. One skilled in the art will recognize that the selection of the exact dose and composition and the most appropriate delivery regimen will also be influenced by *inter alia* the pharmacological properties of the formulation, the nature and severity of the condition being treated, and the physical condition and mental acuity of the recipient, as well as the potency of the specific compound, the age, condition, body weight, sex and response of the patient to be treated, and the stage/severity of the disease, as well as genetic differences between patients.

Administration of active ingredients may be continuous or intermittent (e.g. by bolus injection). The dosage may also be determined by the timing and frequency of administration.

Suitable doses of active ingredients include those referred to in the medical literature, such as Martindale - The Complete Drug Reference (34th Edition) and the documents referred to therein . Suitable doses of active ingredients are therefore in the range of about 0.01 mg/kg of body weight to about 1,000 mg/kg of body weight. More preferred ranges are about 0.1 mg/kg to about 20 mg/kg, such as about 1 mg/kg to about 10 mg/kg on a daily basis, when given orally.

However, suitable doses of pemirolast are known to those skilled in the art. For example suitable lower limits of daily dose ranges are about 2 mg, for example about 5 mg, such as about 10 mg, and more preferably about 20 mg; and suitable upper limits of daily dose ranges are about 200 mg, for example about 100 mg, such as about 80 mg, and more preferably about 60 mg. Daily peroral doses may thus be between about 2 mg and about 50 mg, such as about 5 mg and about 40 mg, and preferably about 10 mg and about 30 mg. Suitable individual doses may be about 20 mg, or about 40 mg, per day. The above doses/dose ranges are all irrespective of whether the formulation employed is a combined preparation or a kit of parts as hereinbefore described.

Similarly, suitable doses of ramatroban are known to those skilled in the art. Peroral doses are therefore in the range of about 0.5 mg to about 400 mg, such as about 2 mg to about 200 mg, preferably about 20 mg to about 150 mg, for example about 80 mg (e.g. 100 mg) to about 150 mg, per day, irrespective of whether the formulation employed is a combined preparation or a kit of parts as hereinbefore described.

In any event, the medical practitioner, or other skilled person, will be able to determine routinely the actual dosage, which will be most suitable for an individual patient. The above-mentioned dosages are exemplary of the average case; there can, of course, be individual instances where higher or lower dosage ranges are merited, and such are within the scope of this invention.

Wherever the word "about" is employed herein, for example in the context of doses of active ingredients, it will be appreciated that such variables are approximate and as such may vary by ± 10%, for example ± 5% and preferably ± 2% (e.g. ±1%) from the numbers specified herein.

The combination product described herein may have the advantage that, in the treatment of the conditions mentioned hereinbefore, they may be more convenient for the physician and/or patient than, be more efficacious than, be less toxic than, have a broader range of activity than, be more potent than, produce fewer side effects than, or that it may have other useful pharmacological properties over, similar treatments known in the prior art for use in the treatment of inflammatory disorders (such as atherosclerosis and associated cardiovascular conditions) or otherwise.

The invention is illustrated by the following examples.

### Examples

### Example 1

### MonoMac-6 Cell Inflammatory Mediator Release Assays

MonoMac-6 (MM6) cells (Ziegler-Heitbrock et al, Int. J. Cancer, 41, 456 (1988)) are cultured (37°C/5% CO₂) in RPMI-1640 medium supplemented with 1 mM sodium pyruvate, 1×nonessential amino acids, 1-100 µg/mL insulin, 1 mM oxalacetic acid, 100 units/mL penicillin, 100 µg/mL streptomycin and 10% (v/v) fetal bovine serum. For differentiation, TGFβ (2 ng/ml) and 1,25(OH)₂D3 (50 nM) are added, generally for about 2-4 days.

To stimulate release of the inflammatory mediator leukotriene B₄ (LTB₄), differentiated or undifferentiated MM6 cells (at 1-15×10⁶/mL; 0.5-1 mL) are incubated for 5-30 minutes (at 37°C in PBS with calcium) with 25-50 µM arachidonic acid and 2-10 µM calcium ionophore A23187 (A23187 may also be used without arachidonic acid). The MM6 cells may also be stimulated with documented biologically active concentrations of adenosine diphosphate (ADP), and/or the thromboxane analogue U-46619, with or without A23187 and/or arachidonic acid as above. The MM6 incubations/stimulations above may also be performed in the presence of human platelets (from healthy donor blood) with an MM6:platelet ratio of 1:10 to 1:10000. The incubations/stimulations are stopped with two volumes of cold methanol and prostaglandin B₂ (PGB₂) added as internal standard. The samples are centrifuged and the supernatants are diluted with water to reach a final methanol concentration of 30% and pH is adjusted to 3-4. Arachidonic acid metabolites in the supernatant are extracted on preconditioned (1 mL methanol followed by 1 mL H₂O) C18 solid phase columns (Sorbent Technology, U.K.). Metabolites are eluted with methanol, whereafter one volume of water is added to the eluate. For reverse phase HPLC, 76 µL of each sample is mixed with 39 µL H₂O (other volume ratios may also be used). A Waters RCM 8×10 column is eluted with methanol/acetonitrile/H₂O/acetic acid (30:35:35:0.01 v/v) at 1.2 mL/min. The absorbance of the eluate is monitored at 270 nm for detection and quantitation of PGB₂ and LTB₄ Commercially available enzyme immuno-assay kits (EIA/ELISA kits) for measuring LTB₄ may also be used according to instructions from the kit manufacturer(s). Using commercially available enzyme immuno-assay kits (EIA/ELISA kits) according to instructions from the manufacturer(s), supernatants from the MM6 incubations/stimulations above may also be analysed with regard to content of the inflammatory mediators prostaglandin E₂ (PGE₂) and/or thromboxane B₂ (TXB₂).

Stock solutions of pemirolast and ramatroban are prepared in ethanol, DMSO, N-methyl-2-pyrrolidone, PEG 400, propylene glycol and/or deionized water or physiological saline solution, with sonication, warming and adjustment of pH as needed (other vehicles may also be used). Cells are incubated (at 37°C/5% CO₂ in PBS without calcium or in RPMI-1640 with 1-10% fetal bovine serum, with or without supplements) with test drug(s) (pemirolast in combination with ramatroban, pemirolast alone and ramatroban alone) for 1 minute to 24 hours prior to MM6 stimulation for inflammatory mediator release (test drug(s) may also be added simultaneously with MM6 stimulation). The drugs are added to reach final concentrations of 1 nM to 100 µM (for comparison, some experiments are performed without the drugs).

To stimulate release of inflammatory cytokines and chemokines such as IL-1β, IL-6, TNF, IL-8, IL-10, IL-12p70, MCP-1, differentiated or undifferentiated MM6 cells (at 1-10×10⁶/mL) are incubated (37°C/5% CO₂) for 4-24 hours (in RPMI-1640 with 1-10% fetal bovine serum, with or without supplements) with lipopolysaccharide (LPS, final concentration 1-100 ng/mL), phorbol-12-myristate-13-acetate (PMA, final concentration 1-100 ng/mL) or an LPS/PMA mixture. The MM6 cells may also be stimulated with documented biologically active concentrations of adenosine diphosphate (ADP), arachidonic acid, calcium ionophore A23187 and/or the thromboxane analogue U-46619, with or without PMA and/or LPS as above. The MM6 cell incubations/stimulations may also be performed in the presence of human platelets (from healthy donor blood) with an MM6:platelet ratio of 1:10 to 1:10000. Cells are incubated (at 37°C/5% CO₂ in RPMI-1640 with 1-10% fetal bovine serum, with or without supplements) with test drug(s) (pemirolast in combination with ramatroban, pemirolast alone and ramatroban alone; as above regarding stock solutions and concentrations) for 1 minute to 24 hours prior to MM6 stimulation (for comparison, some experiments are performed without the drugs; test drug(s) may also be added simultaneously with MM6 stimulation). After spinning down the cells after the incubations/stimulations, human cytokine and chemokine concentrations in the supernatants are quantitated using a Cytometric Bead Array (BD Biosciences Pharmingen, San Diego, USA) according to the manufacturer's instructions. Commercially available enzyme immuno-assay kits (EIA/ELISA kits) for measuring cytokines and chemokines may also be used according to instructions from the manufacturer(s). The cell pellets are stored frozen (-80°C) in RLT buffer (QIAGEN, Valencia, CA) until further processing for microarray experiments (see Example 12 below).

### Example 2

### Human Peripheral Blood Cell Inflammatory Mediator Release Assays

Human peripheral blood mononuclear cells (PBMC) or polymorphonuclear cells (PMN) are isolated by Lymphoprep or Ficoll-Paque separation (with or without Polymorphoprep separation and/or Dextran sedimentation) from healthy donor blood using established protocols.

To stimulate release of the inflammatory mediator leukotriene B₄ (LTB₄), PBMC or PMN (at 1-15×10⁶/mL; 0.5-1 mL) are incubated for 5-30 minutes (at 37°C in PBS with calcium) with 25-50 µM arachidonic acid and 2-10 µM calcium ionophore A23187 (A23187 may also be used without arachidonic acid). The PBMC/PMN may also be stimulated with documented biologically active concentrations of adenosine diphosphate (ADP), and/or the thromboxane analogue U-46619, with or without A23187 and/or arachidonic acid as above. The PBMC/PMN incubations/stimulations above may also be performed in the presence of human platelets (from healthy donor blood) with a PBMC/PMN:platelet ratio of 1:10 to 1:10000. The incubations/stimulations are stopped with two volumes of cold methanol and prostaglandin B₂ added is as internal standard. The samples are centrifuged and the supernatants are diluted with water to reach a final methanol concentration of 30% and pH is adjusted to 3-4. Arachidonic acid metabolites in the supernatant are extracted on preconditioned (1 mL methanol followed by 1 mL H₂O) C18 solid phase columns (Sorbent Technology, U.K). Metabolites are eluted with methanol, after which one volume of water is added to the eluate. For reverse phase HPLC, 76 µL of each sample is mixed with 39 µL H₂O (other volume ratios may also be used). A Waters RCM 8×10 column is eluted with methanol/acetonitrile/H₂O/acetic acid 30:35:35:0.01 v/v) at 1.2 mL/minute. The absorbance of the eluate is monitored at 270 nm for detection and quantitation of PGB₂ and LTB₄. Commercially available enzyme immune-assay kits (EIA/ELISA kits) for measuring LTB₄ may also be used according to instructions from the manufacturer(s). Using commercially available enzyme immuno-assay kits (EIA/ELISA kits) according to instructions from the manufacturer(s), supernatants from the PBMC/PMN incubations/stimulations above may also be analysed with regard to content of the inflammatory mediators prostaglandin E₂ (PGE₂) and/or thromboxane B₂ (TXB₂). Cells are incubated (at 37°C in PBS without calcium or in RPMI-1640 with 0-10% fetal bovine serum) with test drug(s) (pemirolast and ramatroban, pemirolast alone and ramatroban alone) for 1 minute to 24 hours prior to PBMC/PMN stimulation for inflammatory mediator release (see Example 1 above for details regarding drug stock solutions and concentrations; test drug(s) may also be added simultaneously with PBMC/PMN stimulation). For comparison, some experiments are performed without the drugs.

To stimulate release of inflammatory cytokines and chemokines such as IL-1β, IL-6, TNF, IL-8, IL-10, IL-12p70, MCP-1, PBMC/PMN (at 1-10×10⁶/mL) are incubated (37°C/5% CO₂) for 4-24 hours (in RPMI-1640 with 1-10% fetal bovine serum) with lipopolysaccharide (LPS, final concentration 1-100 ng/mL), phorbol-12-myristate-13-acetate (PMA, final concentration 1-100 ng/mL) or an LPS/PMA mixture. The PBMC/PMN cells may also be stimulated with documented biologically active concentrations of adenosine diphosphate (ADP), arachidonic acid, calcium ionophore A23187 and/or the thromboxane analogue U-46619, with or without PMA and/or LPS as above. The PBMC/PMN incubations/stimulations may also be performed in the presence of human platelets (from healthy donor blood) with a PBMC/PMN:platelet ratio of 1:10 to 1:10000. Cells are incubated (at 37°C/5% CO₂ in RPMI-1640 with 1-10% fetal bovine serum) with test drug(s) (pemirolast in combination with ramatroban, pemirolast alone and ramatroban alone, as above) for 1 minute to 24 hours prior to PBMC/PMN stimulation for cytokine/chemokine release (for comparison, some experiments are performed without the drugs; test drug(s) may also be added simultaneously with PBMC/PMN stimulation). After spinning down the cells after the incubations/stimulations, human cytokine and chemokine concentrations in the supernatants are quantitated using a Cytometric Bead Array (BD Biosciences Pharmingen, San Diego, USA) according to the manufacturer's instructions. Commercially available enzyme immuno-assay kits (EIA/ELISA kits) for measuring cytokines and chemokines may also be used according to instructions from the manufacturer(s). The cell pellets are stored frozen (-80°C) in RLT buffer (QIAGEN, Valencia, CA) until further processing for microarray experiments (see Example 12 below).

### Example 3

### Mouse Mast Cell Inflammatory Mediator Release Assays

Bone marrow-derived cultured mouse mast cells (mMCs) are obtained by culturing bone marrow cells from C57BL/6 mice. The bone marrow cells (from mouse femurs flushed with PBS) are cultured (37°C/5% CO₂) in 10% WEHI-3 or X-63 enriched conditioned RPMI 1640, supplemented with 10% heat-inactivated fetal bovine serum, 4 mM L-glutamine, 50 µM 2-mercaptoethanol, 1 mM sodium pyruvate, 0.1 mM non-essential amino acids, 10 mM Hepes, and 100 µg/mL penicillin/streptomycin. Development of mast cells (which grow in suspension) is confirmed by expression of Kit (by flow-cytometry) on the cell surface and/or by toluidine blue staining (generally after at least 3-5 weeks of culture).

Bone marrow-derived cultured mouse mast cells of connective tissue type (CT-type) are obtained by culturing bone marrow cells from C57BL/6 mice. The bone marrow cells are cultured (37°C/5% CO₂) in RPMI-1640 medium containing 10% filtered FCS, 4 mM L-glutamine, 1 mM sodium pyruvate, 100 IU/mL penicillin G, 100 µg/mL streptomycin, 0.1 mM MEM non-essential amino acids and 50 µM 2-ME, supplemented with 50 ng/mL recombinant murine stem cell factor and 1 ng/mL murine recombinant IL-4. Mast cell development is confirmed by expression of Kit (by flow-cytometry) on the cell surface and/or by toluidine blue staining (generally after at least 3-5 weeks of culture).

Mouse mast cell lines MC/9 (obtained from ATCC, Product no CRL-8306) and C1.MC/C57.1 (Young et al., Proc. Natl. Acad. Sci. USA, 84, 9175 (1987)) may also be used. The MC/9 cells are cultured according to instructions from ATCC (http://www.atcc.org), and C1.MC/C57.1 cells are cultured as described in Rumsaeng et al (J. Immunol. 158, 1353 (1997)).

For activation/stimulation through cross-linking of the IgE-receptor, the cultured mast cells are initially sensitized for 90 minutes at 37°C (5% CO₂) with a monoclonal mouse anti-TNP IgE-antibody (IgE1-b4, ATCC, Rockville, MD, USA), used as a 15% hybridoma supernatant. Cells to be used in the N-acetylbeta-D-hexosaminidase (or histamine) or cytokine/chemokine release assays (see below) are then subjected to two washings with PBS and re-suspended in RPMI-1640 medium supplemented with 0.2% bovine serum albumin (BSA) (Sigma) before the cells (at 0.5-10x10⁶/mL) are activated by addition of 100 ng/mL TNP-BSA (Biosearch Technologies, San Francisco, CA) with a coupling ratio of 9/1. The incubation (37°C/5% CO₂) with TNP-BSA is 30 minutes for the analysis of beta-hexosaminidase (or histamine) release and 6-24 hours for analysis of cytokine and chemokine release. Cells are incubated (37°C/5% CO₂) with test drug(s) (pemirolast and ramatroban, pemirolast alone and ramatroban alone) for 1 minute to 24 hours prior to addition of TNP-BSA (see Example 1 above for detail regarding drug stock solutions and concentrations; test drug(s) may also be added simultaneously with TNP-BSA stimulation). For comparison, some experiments are performed without the drugs. After the incubations/stimulations, the samples are centrifuged and the supernatants analysed with regard to content of beta-hexosaminidase (or histamine) and/or cytokines/chemokines as described below. The cell pellets are stored frozen (-80°C) in RLT buffer (QIAGEN, Valencia, CA) until further processing for microarray experiments (see Example 12 below).

For detection of IgE-dependent release of the granular mast cell enzyme beta-hexosaminidase, an enzymatic colourimetric assay is used. 60 µL from each well supernatant is transferred to a 96 well plate and mixed with an equal volume of substrate solution (7.5 mM p-nitrophenyl-N-acetyl-b-D-glucosaminide dissolved in 80 mM citric acid, pH 4.5). The mixture is incubated on a rocker platform for 2 hours at 37°C. After incubation, 120 µL of glycine (0.2 M, pH 10.7) is added to each well and the absorbance at 405 and 490 nm is measured using an Emax Precision Microplate Reader (Molecular Devices, Sunnyvale, CA). Release of beta-hexosaminidase is expressed as a percentage of total beta-hexosaminidase determined after cell lysis. For detection of IgE-dependent release of granular mast cell histamine, commercially available enzyme immuno-assay kits (EIA/ELISA kits) for measuring histamine is used according to instructions from the manufacturer(s).

For detection of IgE-dependent release of mouse mast cells cytokines and chemokines such as IL-6, IL-4, TNF, IL-1β, KC, MCP-1, IL-1.0, IL-12p70, IFNγ, a Cytometric Bead Array (BD Biosciences Pharmingen, San Diego, USA) is used according to the manufacturer's instructions. Commercially available enzyme immuno-assay kits (EIA/ELISA kits) for measuring cytokines and chemokines may also be used according to instructions from the manufacturer(s).

In addition to the mast cell experiments above, mast cell-inhibiting effects of the test drug(s) (as above) may also be studied using well established and documented experimental approaches and assays for analysing induced (with e.g. anti-IgE (with or without pre-treatment of the cells with rat or mouse IgE), concanavalin A, protein L, compound 48/80, ionophore A23187, PMA) release of histamine, beta-hexosaminidase or tryptase from freshly isolated peritoneal rat or mouse mast cells.

### Example 4

### RAW 264.7 Cell Inflammatory Mediator Release Assays

RAW 264.7 cells are cultured (37°C/5% CO₂) in DMEM, supplemented with 100 units /mL penicillin, and 100 µg/mL streptomycin and 10% fetal bovine serum.

To stimulate release of inflammatory cytokines and chemokines such as IL-6, TNF, IL-1β, KC, MCP-1, IL-10, IL-12p70, IFNγ, RAW 264.7 cells (at 1-10×10⁶/mL) are incubated (37°C/5% CO₂) for 4-24 hours (in DMEM with 1-10% fetal bovine serum, with or without supplements) with lipopolysaccharide (LPS, final concentration 1-100 ng/mL), phorbol-12-myristate-13-acetate (PMA, final concentration 1-100 ng/mL) or an LPS/PMA mixture. The RAW 264.7 cells may also be stimulated with documented biologically active concentrations of adenosine diphosphate (ADP), arachidonic acid, calcium ionophore A23187 and/or the thromboxane analogue U-46619, with or without PMA and/or LPS as above. The RAW 264.7 incubations/stimulations may also be performed in the presence of mouse or human (from healthy donor blood) platelets with a RAW 264.7:platelet ratio of 1:10 to 1:10000. Cells are incubated (at 37°C/5% CO₂ in DMEM with 1-10% fetal bovine serum, with or without supplements) with test drug(s) (pemirolast and ramatroban, pemirolast alone and ramatroban alone) for 1 minute to 24 hours prior to RAW 264.7 stimulation for cytokine/chemokine release (see Example 1 above for details regarding drug stock solutions and concentrations; test drug(s) may also be added simultaneously with RAW 264.7 stimulation). For comparison, some experiments are performed without the drugs. After spinning down the cells after the incubations/stimulations, mouse cytokine and chemokine concentrations in the supernatants are quantitated using a Cytometric Bead Array (BD Biosciences Pharmingen, San Diego, USA) according to the manufacturer's instructions. Commercially available enzyme immuno-assay kits (EIA/ELISA kits) for measuring cytokines and chemokines may also be used according to instructions from the manufacturer(s). The cell pellets are stored frozen (-80°C) in RLT buffer (QIAGEN, Valencia, CA) until further processing for microarray experiments (see Example 12 below).

### Example 5

### Rat Paw Inflammation Induced by Carrageenan

This assay is essentially according to that described by Winter et al (Proc. Soc. Exp. Biol. Med, 111, 544 (1962)). Test drug(s) (pemirolast and ramatroban, pemirolast alone and ramatroban alone) at doses of 0.03 to 50 mg/kg are administered subcutaneously, intravenously, intraperitoneally or orally every 2-24 hours to male Sprague-Dawley or Wistar rats weighing approximately 150-400 g (for comparison, some experiments are performed without the drugs). Prior to administration, stock solutions of drugs (see Example 1 above) are diluted as needed in e.g. 0.5% or 1% methylcellulose in water (for oral treatment) or saline (for parenteral administration). Other vehicles may also be used. 1 minute to 24 hours after the first drug dose, a 0.5, 1.0 or 2.0% solution of carrageenan (Type IV Lambda, Sigma Chemical Co.) in 0.9% saline is injected into the subplantar region of one hind paw of anaesthetised rats. Before, and at indicated intervals 3-24 hours after carrageenan injection, the volume of the injected paw is measured with a displacement plethysmometer connected to a pressure transducer with a digital indicator. The degree of swelling indicates the degree of inflammatory edema. 3-24 hours after carrageenan injection, the rats are sacrificed and perfused with saline or PBS (other perfusion media may also be used). Plantar soft tissue biopsies from the inflamed paws are collected, weighed, stored frozen (samples for microarray analysis are frozen at -80°C in TRIzol, Invitrogen, Carlsbad, CA), and, as described below (Example 10 and 12), subsequently analyzed with regard to 1) myeloperoxidase (MPO) accumulation, reflecting inflammatory neutrophil leukocyte accumulation; and/or 2) tissue gene expression using microarray technology. Non-inflamed paw tissue from untreated rats provides base-line levels of MPO and gene expression. Tissue inflammation may also be studied using conventional histological and immunohistochemical techniques. Paw inflammation may also be induced by subplantar injection of compound 48/80 (48/80, 1-5 µg in 50-100 µl PBS or saline) (instead of carrageenan), followed by measurement of inflammatory paw swelling and collection of tissue biopsies for microarray and/or MPO analysis (as above) 30 min to 8 hours after 48/80 injection.

### Example 6

### Mouse Ear Inflammation Induced by Croton Oil

This assay is essentially according to that described by Tonelli et al (Endocrinology 77, 625 (1965)) (other strains of mice may also be used). Test drug(s) (pemirolast and ramatroban, pemirolast alone and ramatroban alone) at doses of 0.03 to 50 mg/kg are administered subcutaneously, intravenously, intraperitoneally or orally every 2-24 hours (for comparison, some experiments are performed without the drugs). Prior to administration, stock solutions of drugs (see Example 1 above) are diluted as needed in e.g. 0.5% or 1% methylcellulose in water (for oral treatment) or saline (for parenteral administration). Other vehicles may also be used. 1 minute to 24 hours after the first drug dose, 10-30 µL of a 2.0 or 4.0% solution of croton oil in acetone or ethanol is applied topically to one or both ears. At indicated intervals 4-12 hours after croton oil application, the animals are sacrificed, and punch biopsies of the ears are weighed to determine the inflammatory swelling of the ears (ear thickness may also be measured to determine the swelling). The biopsies from the inflamed ears are collected, stored frozen (samples for microarray analysis are frozen at -80°C in TRIzol), and, as described below (Example 10 and 12), subsequently analyzed with regard to 1) myeloperoxidase (MPO) accumulation, reflecting inflammatory neutrophil leukocyte accumulation; and/or 2) tissue gene expression using microarray technology. Non-inflamed ear biopsies from untreated mice provide base-line levels of swelling, MPO and gene expression. Tissue inflammation may also be studied using conventional histological and immunohistochemical techniques.

### Example 7

### Mouse Ear Inflammation Induced by Phorbol Ester or Arachidonic Acid

These assays are essentially according to those described by Chang et al (Eur. J. Pharmacol. 142, 197 (1987)) (although other strains of mice may also be used). Test drug(s) (pemirolast and ramatroban, pemirolast alone and ramatroban alone) at doses of 0.03 to 50 mg/kg are administered subcutaneously, intravenously, intraperitoneally or orally every 2-24 hours to male or female mice (for comparison, some experiments are performed without the drugs). Prior to administration, stock solutions of drugs (see Example 1 above) are diluted as needed in e.g. 0.5% or 1% methylcellulose in water (for oral treatment) or saline (for parenteral administration). Other vehicles may also be used. 1 minute to 24 hours after the first drug dose, 1-10 µg of phorbol 12-myristate 13-acetate (PMA), tetradecanoyl phorbol acetate (TPA), or 1-5 mg arachidonic acid in 10-30 µl acetone or ethanol is applied topically to one or both ears. 4-12 hours after PMA or TPA application, and 30 min to 6 hours after arachidonic acid application, the animals are sacrificed, and punch biopsies of the ears are weighed to determine the inflammatory swelling of the ears (ear thickness may also be measured to determine the swelling). The biopsies from the inflamed ears are collected, stored frozen (samples for micro array analysis are frozen at -80°C in TRIzol), and, as described below (Example 10 and 12), subsequently analyzed with regard to 1) myeloperoxidase (MPO) accumulation, reflecting inflammatory neutrophil leukocyte accumulation; and/or 2) tissue gene expression using microarray technology. Non-inflamed ear biopsies from untreated mice provide base-line levels of swelling, MPO and gene expression. Tissue inflammation may also be studied using conventional histological and immunohistochemical techniques.

### Example 8

### Acute Tissue Reaction and Inflammation in Response to Injury in Mouse and Rat

Male CBA or NMRI mice weighing approximately 15-30 g, or male Wistar or Sprague-Dawley rats weighing approximately 150-450 g, are used (other strains of mice and rats may also be used). Acute tissue injury and acute inflammation is achieved in the distal part of the tail or one of the ears using a scalpel under aseptic conditions. One, two or three parallel, approximately 5-15 mm long, longitudinal cuts are made through all layers of the skin. Test drug(s) (pemirolast and ramatroban, pemirolast alone and ramatroban alone) at doses of 0.03 to 50 mg/kg are administered subcutaneously, intravenously, intraperitoneally or orally every 2-24 hours, with the first dose given 1 minute to 24 hours before tissue injury (for comparison, some experiments are performed without the drugs). Prior to administration, stock solutions of drugs (see Example 1 above) are diluted as needed in e.g. 0.5% or 1% methylcellulose in water (for oral treatment) or saline (for parenteral administration). Other vehicles may also be used. 2-48 hours after injury, the animals are killed and the injured segments of the tissues are removed, weighed and stored frozen (samples for microarray analysis are frozen at -80°C in TRIzol, and, as described below (Example 10 and 12), subsequently analyzed with regard to 1) myeloperoxidase (MPO) accumulation, reflecting inflammatory neutrophil leukocyte accumulation; and/or 2) tissue gene expression using microarray technology. Corresponding non-injured/non-inflamed tissues from untreated animals provide base-line levels of MPO and gene expression. Tissue reactions and inflammation in response to injury may also be studied using conventional histological and immunohistochemical techniques.

### Example 9

### Acute Tissue Reaction and Inflammation in Response to Injury in Rat

Male Sprague-Dawley rats weighing 350-500 g are used (although other strains of rats may also be used). Animals are anesthetized with Isoflurane in oxygen and acute tissue injury and acute inflammation is achieved in the left common carotid artery as follows: After surgical exposure of the left common, external and internal carotid arteries and temporary cessation of local blood flow with temporary ligatures, a balloon catheter (2-French Fogarty) is passed through the external carotid into the aorta. Next, the balloon is inflated with sufficient water to distend the common carotid artery and then pulled back to the external carotid. This procedure is repeated three times, and then the catheter is removed, the external carotid ligated and the wound closed. Test drug(s) (pemirolast and ramatroban, pemirolast alone and ramatroban alone) at doses of 0.03 to 50 mg/kg are administered subcutaneously, intravenously, intraperitoneally or orally every 2-24 hours, with the first dose given 1 minute to 24 hours before tissue injury (for comparison, some experiments are performed without the drugs). Prior to administration, stock solutions of drugs (see Example 1 above) are diluted as needed in e.g. 0.5% or 1% methylcellulose in water (for oral treatment) or saline (for parenteral administration). Other vehicles may also be used. 2-48 hours after injury, the animals are anesthetized with Isoflurane in oxygen and their left carotid arteries exposed. Clamps are put on the very proximal part of the common and internal carotid arteries, respectively, and then the vessel between the clamps is gently flushed with sterile saline and/or TRIzol, removed, weighed and stored frozen (samples for microarray analysis are frozen at -80°C in TRIzol), and, as described below (Example 10 and 12), subsequently analyzed with regard to 1) myeloperoxidase (MPO) accumulation, reflecting inflammatory neutrophil leukocyte accumulation; and/or 2) tissue gene expression using microarray technology. Corresponding non-injured/inflamed vessels from untreated rats provide base-line levels of MPO and gene expression. Tissue reactions and inflammation in response to injury may also be studied using conventional histological and immunohistochemical techniques.

### Example 10

### Inflammatory Accumulation of Tissue Myeloperoxidase

The enzyme myeloperoxidase (MPO) is abundant in neutrophil leukocytes and is often used as a marker for the detection of neutrophil accumulation in inflamed tissue. To determine inflammatory myeloperoxidase accumulation in inflamed mouse and rat tissues (as described in Example 5-9 above), the tissues are homogenised in 0.5% hexadecyltrimethyl-ammonium bromide, and freeze-thawed. The MPO activity of the supernatant is determined spectrophotometrically as the change in absorbance at 650 nm (25°C) occurring in the redox reaction of H₂O₂-tetramethylbenzidine catalysed by MPO. Values are expressed as MPO units/mg tissue.

### Example 11

### Smooth Muscle Cell Assays

Rat aortic smooth muscle cells (RASMCs) are isolated as previously described (Hedin et al, Arterioscler. Thromb. Vasc. Biol., 17, 1977 (1997)). Cells are cultured (37°C/5% CO₂) in Ham's medium F-12 supplemented with 10% fetal bovine serum, 50 µg/mL L-ascorbic acid, 50 µg/mL streptomycin, 50 IU/mL penicillin (F-12/10% fetal bovine serum), grown to confluence, serially passaged by trypsinization, and used in experiments after 2-6 passages. RASMCs are seeded in 24-well plates at a density of approximately 4x10⁴ cells per well in F-12/10% fetal bovine serum (plates with larger numbers of wells per plate and appropriate lower numbers of cells per well may also be used). After 24 hours, the cells are synchronized in G0/G1 phase by starvation in Ham's medium F-12 supplemented with 0.1% bovine serum albumin (BSA), 50 µg/mL L-ascorbic acid, 50 µg/mL streptomycin and 50 IU/mL penicillin (F-12/0.1% BSA) for 24-48 hours. To estimate DNA synthesis, starved RASMCs are stimulated with either 10 ng/ml IGF-1 or 10% fetal bovine serum for 12-48 hours (other well established mitogens such as PDGF may also be used). Test drug(s) (pemirolast and ramatroban, pemirolast alone and ramatroban alone) are added 1 minute to 24 hours prior to stimulation (see Example 1 above for details regarding drug stock solutions and concentrations; test drug(s) may also be added simultaneously with stimulation). For comparison, some experiments are performed without the drugs. The cells are labelled with 1 µCi [3H]-thymidine for 8 hours before the end of the stimulation period. The plates are then washed with ice-cold PBS, incubated overnight with ice-cold 10% (w/v) trichloroacetic acid, lysed in 0.2 M sodium hydroxide, and radioactivity is measured in a liquid scintillation counter. The stimulated RASMC proliferation may also be analyzed using commercially available bromodeoxyuridine (BrdU) cell proliferation assays (for example Cell Proliferation ELISA, BrdU, from Roche Applied Science), the cell proliferation reagent WST-1 (Roche Diagnostics Scandinavia AB, Bromma, Sweden) (both according to the manufacturer's instructions), or by cell counting. In separate experiments (to study gene expression), larger numbers of starved RASMCs (1-5 x 10⁶ cells per well) are stimulated with 10 ng/ml IGF-1 or 10% fetal bovine serum (or PDGF) as above, or with LPS (1-100 ng/mL), with 1-10% fetal bovine serum for 4-48 hours (all stimuli with and without test drug(s) as above). The cells are then collected and stored frozen (-80°C) in RLT buffer (QIAGEN, Valencia, CA) until further processing for microarray experiments (see Example 12 below).

Human bronchial smooth muscle cells (HBSMCs, Promocell, Heidelberg, Germany) are cultured in DMEM supplemented with 10% FBS, 100 units/mL penicillin, 100 µg/mL streptomycin, 0.12 IU/mL insulin, and with or without 2 µg/mL amphotericin B. Prior to the experiments, cells may be growth arrested for 24 hours in low-FBS (0.3-5%), insulin-free medium. To stimulate formation and release of inflammatory cytokines and chemokines such as IL-8 and eotaxin, HBSMCs (at 80% confluence, corresponding to approximately 8×10⁵/25 cm² flask) are incubated (37°C/5% CO₂) for 24-48 hours (in DMEM with 1-10% fetal bovine serum, with or without supplements) with different combinations of IL-1β and TNF-α (both at 1-50 ng/mL). Cells are incubated (at 37°C/5% CO₂ in DMEM with 0.3-10% fetal bovine serum, with or without supplements) with test drug(s) (pemirolast in combination with ramatroban, pemirolast alone and ramatroban alone, as above) for 1 minute to 24 hours prior to HBSMC stimulation (see Example 1 above for details regarding drug stock solutions and concentrations; test drug(s) may also be added simultaneously with HBSMC stimulation). For comparison, some experiments are performed without the drugs. After the incubations/stimulations, human cytokine and chemokine concentrations in the supernatants are quantitated using commercially available enzyme immuno-assay kits (EIA/ELISA kits) according to instructions from the manufacturer(s). The cells are then collected and stored frozen (-80°C) in RLT buffer (QIAGEN, Valencia, CA) until further processing for microarray experiments (see Example 12 below).

### Example 12

### Analysis of Gene Expression

Total RNA from mouse and rat tissues (see Example 5 to 9, 15 and 16) is isolated using TRIzol (Invitrogen, Carlsbad, CA) followed by RNeasy cleanup (QIAGEN, Valencia, CA) according to manufacturers' protocols. Total RNA from the cell incubations/stimulations described in the examples above and below (mouse mast cells, MonoMac-6, PBMC, PMN, RAW 264.7, RASMC, HBSMC, NB4, HL-60) is isolated using RNeasy Mini Kit (QIAGEN), with or without RNase-Free DNase set (QIAGEN), according to the manufacturer's protocol(s). Depending on the species from which the different tissues and cells originate, microarray analysis is performed using GeneChip® Human Genome U133 Plus 2.0 Array, GeneChip® Mouse Genome 430 2.0 Array or GeneChip® Rat Genome 230 2.0 Array, or corresponding newer version of these chips (all arrays from Affymetrix, Santa Clara, CA) according to the manufacturer's protocols. The microarray expression data is analyzed using e.g. GeneChip Operating Software (Affymetrix) and Bioconductor/R (www.bioconductor.org). Other relevant software may also be used.

Gene expression from the different species may also be analyzed using Human Genome Survey Microarray V2.0, Mouse Genome Survey Microarray V2.0 or Rat Genome Survey Microarray (or corresponding newer version of these arrays) according to protocols from the manufacturer Applied Biosystems (Foster City, CA). These microarray expression data are analyzed using e.g. 1700 Chemiluminescent Microarray Analyzer (Applied Biosystems, Foster City, CA) supplied with an Coracle® database of annotations, GeneSpring 7.2 (Agilent Technologies, Inc., Palo Alto, CA) and Microarray Suite version 5.0 software (MAS 5.0, Affymetrix). Other relevant software may also be used.

Gene expression (mRNA levels) may also be analyzed using quantitative or semiquantitative PCR. Analysis of gene expression at the protein level may be analyzed using commercially available enzyme immuno-assay kits (ETA/ELISA kits) (according to instructions from the manufacturer(s)), or conventional Western blot and/or immunohistochemical approaches.

### Example 13

### Cell Proliferation Assays

Proliferation of stimulated and unstimulated mouse mast cells, MonoMac-6 cells, RAW 264.7 cells, NB4 cells, HL-60 cells and HBSMC described in the examples above and below (with or without growth arrest for 24-48 hours in 0.1-5% fetal bovine serum prior to the addition of the respective test drugs and/or stimuli described in the Examples above and below for 24-72 hours) is measured using the cell proliferation reagent WST-1 (Roche Diagnostics Scandinavia AB, Bromma, Sweden) or commercially available bromodeoxyuridine (BrdU) cell proliferation assays (for example Cell Proliferation ELISA, BrdU, from Roche Applied Science) according to the manufacturers' instructions. Other conventional tests of cell proliferation may also be used.

### Example 14

### Platelet Aggregation Tests

Aggregation of rabbit or human platelets (in platelet rich plasma or whole blood) induced by adenosine diphosphate (ADP), arachidonic acid, collagen or the thromboxane analogue U-46619 is analyzed using aggregometry, for example as described by Bertele et al (Eur. J. Pharmacol. 85, 331 (1982)). Induced (as above) platelet aggregation may also be analyzed using washed human or rabbit platelets and/or with other established aggregometry or other corresponding methods for measuring platelet aggregation. Test drug(s) (pemirolast and ramatroban, pemirolast alone and ramatroban alone) are added 1-120 minutes prior to induction of platelet aggregation (see Example 1 above for details regarding drug stock solutions and concentrations; test drug(s) may also be added simultaneously with induction of platelet aggregation). For comparison, some experiments are performed without the drugs.

### Example 15

### Mouse Peritoneal Inflammation-Induced by Zymosan and Other Stimuli

This assay is essentially according to Rao et al (J. Pharmacol. Exp. Ther. 269, 917 (1994)) (other strains of mice may also be used). Test drug(s) (pemirolast and ramatroban, pemirolast alone and ramatroban alone) at doses of 0.03 to 50 mg/kg are administered subcutaneously, intravenously, intraperitoneally or orally every 2-24 hours to the animals (for comparison, some experiments are performed without the drugs). Prior to administration, stock solutions of drugs (see Example 1 above) are diluted as needed in e.g. 0.5% or 1% methylcellulose in water (for oral treatment) or saline (for parenteral administration). Other vehicles may also be used. 1 minute to 24 hours after the first drug dose, 0.5-2 mg zymosan A (Sigma, cat. no. Z4250) in 0.5-1 mL sterile PBS (sonicated and well mixed) is injected intraperitoneally (instead of using zymosan A, peritoneal inflammation may also be induced by intraperitoneal injection of pro-inflammatory concentrations of other well established pro-inflammatory stimuli such as antimouse-IgE (with or without intraperitoneal pretreatment with mouse IgE for 1-3 days), concanavalin A, carrageenan, proteose peptone, LPS, PMA, thioglycolate, arachidonic acid, fMLP, TNF, IL-1β. Test drug(s) may also be administered simultaneously with intraperitoneal injection of zymosan or the other pro-inflammatory stimuli). 2-24 hours after injection of zymosan (or one or more of the other pro-inflammatory stimuli), the animals are sacrificed. The peritoneal cavity is then flushed with 1-3 mL of a lavage buffer (ice-cold PBS with or without 3-5 mM EDTA or 5-10 units/mL heparin). Total and differential leukocyte counts in the lavage fluid are done with a hemocytometer following staining with Türk's solution and/or in cytospin preparations stained with May-Grunwald Giemsa or a modified Wright's (Diff-Quik) stain, respectively, by light microscopy using standard morphological criteria. Other established methods for determining total and differential leukocyte counts may also be used. The remaining lavage fluid is centrifuged (300-3000 x g, 4°C, 3-10 min), and cell-free lavage fluid supernatant is stored frozen (-20°C to -80°) until analyzed for content of inflammatory mediators LTB₄, PGE₂, TXB₂ and/or mouse cytokines/chemokines (e.g. IL-4, IL-6, TNF, IL-1β, KC, MCP-1, IL-10, IL-12p70, IFNγ) content as described in Example 1 and 4 above. The histamine content in the lavage fluid supernatant is determined by using commercially available histamine enzyme immuno-assay kits (EIA/ELISA kits) according to instructions from the manufacturer(s). Inflammatory peritoneal cell activation may also be studied by measuring beta-hexosaminidase activity in the lavage fluid using the beta-hexosaminidase assay described in Example 3. The cell pellets of the lavage fluid are resuspended in 0.1-1.0 mL 0.05 M KHPO₄ pH 6.0 with 0.5% HTAB and stored frozen (-20°C to -80°) until analysis of myeloperoxidase (MPO) content as described by Rao et al (J. Pharmacol. Exp. Ther. 269, 917-25 (1994)). Identical cell pellets from separate animals are stored frozen (-80°C) in RLT buffer (QIAGEN, Valencia, CA) until further processing for microarray experiments (see Example 12). At the time of flushing the peritoneal cavity with lavage buffer, tissue (peritoneal wall, intestines and/or other intra- or retroperitoneal organs/tissues) biopsies from the inflamed peritoneal cavity are collected, weighed, stored frozen (samples for microarray analysis are frozen at -80°C in TRIzol, Invitrogen, Carlsbad, CA), and, as described in Example 12, subsequently analyzed with regard to tissue gene expression using microarray technology. Non-inflamed peritoneal cavities from untreated animals provide base-line levels of MPO, inflammatory mediators, cytokines/chemokines and gene expression. Tissue inflammation may also be studied using conventional histological and immunohistochemical techniques.

### Example 16

### Rat Peritoneal Inflammation-Induced by Zymosan and Other Stimuli

Male Wistar or Sprague Dawley rats weighing approximately 150-450 g are used. Test drug(s) (pemirolast and ramatroban, pemirolast alone and ramatroban) at doses of 0.03 to 50 mg/kg are administered subcutaneously, intravenously, intraperitoneally or orally every 2-24 hours to the animals (for comparison, some experiments are performed without the drugs). Prior to administration, stock solutions of drugs (see Example 1 above) are diluted as needed in e.g. 0.5% or 1% methylcellulose in water (for oral treatment) or saline (for parenteral administration). Other vehicles may also be used. 1 minute to 24 hours after the first drug dose, 1-100 mg zymosan A (Sigma, cat. no. Z4250) in 1-10 mL sterile PBS (sonicated and well mixed) is injected intraperitoneally (instead of using zymosan A, peritoneal inflammation may also be induced by intraperitoneal injection of pro-inflammatory concentrations of other well established pro-inflammatory stimuli such as anti-rat-IgE (with or without intraperitoneal pretreatment with rat IgE for 1-3 days), concanavalin A, protein L, compound 48/80, carrageenan, proteose peptone, LPS, PMA, thioglycolate, arachidonic acid, fMLP, TNF, IL-1β. Test drug(s) may also be administered simultaneously with intraperitoneal injection of zymosan or the other pro-inflammatory stimuli). 2-24 hours after injection of zymosan (or one or more of the other stimuli), the animals are sacrificed. The peritoneal cavity is then flushed with 10-20 ml of a lavage buffer (e.g. ice-cold PBS with or without 3-5 mM EDTA or 5-10 units/mL heparin). Total and differential leukocyte counts in the lavage fluid are done with a hemocytometer following staining with Türk's solution and/or in cytospin preparations stained with May-Grunwald Giemsa or a modified Wright's (Diff-Quik) stain, respectively, by light microscopy using standard morphological criteria. Other established methods for determining total and differential leukocyte counts may also be used. The remaining lavage fluid is centrifuged (300-3000 x g, 4°C, 3-10 min), and cell-free lavage fluid supernatant is stored frozen (-20°C to -80°) until analyzed for content of the inflammatory mediators LTB₄, PGE₂, TXB₂ and/or rat cytokines/chemokines (e.g. IL-4, IL-6, TNF, IL-1β, KC, MCP-1, IL-10, IL-12p70, IFNγ) essentially as described in Example 1 and 4 above. The histamine content in the lavage fluid supernatant is determined by using commercially available histamine enzyme immuno-assay kits (EIA/ELISA kits) according to instructions from the manufacturer(s). Inflammatory peritoneal cell activation may also be studied by measuring beta-hexosaminidase activity in the lavage fluid using the beta-hexosaminidase assay described in Example 3. The cell pellets of the lavage fluid are resuspended in 0.1-1.0 mL 0.05 M KHPO₄ pH 6.0 with 0.5% HTAB and stored frozen (-20°C to -80°) until analysis of myeloperoxidase (MPO) content basically as described by Rao et al (J. Pharmacol. Exp. Ther., 269, 917-25 (1994)). Identical cell pellets from separate animals are stored frozen (-80°C) in RLT buffer (QIAGEN, Valencia, CA) until further processing for microarray experiments (see Example 12). At the time of flushing the peritoneal cavity with lavage buffer, tissue (peritoneal wall, intestines and/or other intra- or retroperitoneal organs/tissues) biopsies from the inflamed peritoneal cavity are collected, weighed, stored frozen (samples for microarray analysis are frozen at -80°C in TRIzol, Invitrogen, Carlsbad, CA), and, as described in Example 12, subsequently analyzed with regard to tissue gene expression using microarray technology. Non-inflamed peritoneal cavities from untreated animals provide base-line levels of MPO, inflammatory mediators, cytokines/chemokines and gene expression. Tissue inflammation may also be studied using conventional histological and immunohistochemical techniques.

### Example 17

### NB4 and HL-60 Cell Inflammatory Mediator Release Assays

Human NB4 cells (Lanotte et al, Blood, 77, 1080 (1991)) are cultured (37°C/5% CO2) in RPMI-1640 medium supplemented with 100 units/mL penicillin, 100 µg/mL streptomycin and 10% (v/v) fetal bovine serum. For differentiation, 1-5 µM all-trans-retinoic acid (ATRA) is added, generally every third day.

Human HL-60 cells (Steinhilber et al, Biochim. Biophys. Acta 1178, 1 (1993)) are cultured (37°C/5% CO2) in RPMI-1640 medium supplemented with 100 units/niL penicillin, 100 µg/mL streptomycin and 10-20% (v/v) fetal bovine serum. For differentiation ATRA (1-5 µM), DMSO (1-2%), PMA (100-500 ng/mL) or vitamin D3 (1-15 µM) is added for 5 days.

To stimulate formation and release of the inflammatory mediator leukotriene B₄ (LTB₄), differentiated or undifferentiated NB4 or HL-60 cells (at 1-15×10⁶/mL) are incubated for 5-30 minutes (at 37°C in PBS with calcium) with 10-40 µM arachidonic acid and/or 2-10 µM calcium ionophore A23187. The NB4 and HL-60 cells may also be stimulated with documented biologically active concentrations of adenosine diphosphate (ADP), fMLP, and/or the thromboxane analogue U-46619, with or without A23187 and/or arachidonic acid as above. The NB4 and HL-60 incubations/stimulations above may also be performed in the presence of human platelets (from healthy donor blood) with an NB4/HL-60:platelet ratio of 1:10 to 1:10000. The incubations/stimulations are stopped with 1 mL cold methanol and prostaglandin B₂ (PGB₂) added as internal standard. The samples are centrifuged and the supernatants are diluted with water to reach a final methanol concentration of 30% and pH is adjusted to 3-4. Arachidonic acid metabolites in the supernatant are extracted on preconditioned (1 mL methanol followed by 1 mL H₂O) C18 solid phase columns (Sorbent Technology, U.K.). Metabolites are eluted with methanol, whereafter one volume of water is added to the eluate. For reverse phase HPLC, 76 µL of each sample is mixed with 39 µL H₂O (other volume ratios may also be used). A Waters RCM 8×10 column is eluted with methanol/acetonitrile/H₂O/acetic acid (30:35:35:0.01 v/v) at 1.2 mL/min. The absorbance of the eluate is monitored at 270 nm for detection and quantitation of PGB₂ and LTB₄. Commercially available enzyme immuno-assay kits (EIA/ELISA kits) for measuring LTB₄ may also be used according to instructions from the kit manufacturer(s). Using commercially available enzyme immune-assay kits (EIA/ELISA kits) according to instructions from the manufacturer(s), the supernatants from the NB4/HL-60 incubations/stimulations above may also be analysed with regard to content of the inflammatory mediators prostaglandin E₂ (PGE₂) and/or thromboxane B₂ (TXB₂). Cells are incubated (at 37°C in PBS without calcium or in RPMI-1640 with 1-20% fetal bovine serum, with or without supplements) with test drug(s) (pemirolast and ramatroban, pemirolast alone and ramatroban alone) for 1 minute to 24 hours prior to NB4 or HL-60 stimulation for inflammatory mediator release (see Example 1 above for details regarding drug stock solutions and concentrations; test drug(s) may also be added simultaneously with NB4/HL-60 stimulation). For comparison, some experiments are performed without the drugs.

To stimulate formation and release of inflammatory cytokines, chemokines and mediators such as IL-1β, IL-6, TNF, IL-8, IL-10, IL-12p70, MCP-1, PAF, C5a, differentiated or undifferentiated NB4 or HL-60 cells (at 1-10×10⁶/mL) are incubated (37°C, 5% CO2) for 4-24 hours (in RPMI-1640 with 1-10% fetal bovine serum, with or without supplements) with lipopolysaccharide (LPS 1-100 ng/mL), phorbol-12-myristate-13-acetate (PMA 1-100 ng/mL) or calcium ionophore A23187 (1-10 µM), or combinations of these stimuli. The NB4 and HL-60 cells may also be stimulated with documented biologically active concentrations of adenosine diphosphate (ADP) and/or the thromboxane analogue U-46619, with or without LPS, PMA and/or A23187 as above. The NB4 and HL-60 incubations/stimulations may also be performed in the presence of human platelets (from healthy donor blood) with an NB4/HL-60:platelet ratio of 1:10 to 1:10000. Cells are incubated (at 37°C, 5% CO₂ in RPMI-1640 with 1-10% foetal bovine serum, with or without supplements) with test drug(s) (pemirolast in combination with ramatroban, pemirolast alone and ramatroban alone, as above) for 1 minute to 24 hours prior to NB4 or HL-60 stimulation for cytokine/chemokine/mediator release (for comparison, some experiments are performed without the drugs; test drug(s) may also be added simultaneously with NB4/HL-60 stimulation). After spinning down cells, human cytokine/chemokine and mediator concentrations in.the supernatants are quantitated using a Cytometric Bead Array (BD Biosciences Pharmingen, San Diego, USA) according to the manufacturer's instructions. Commercially available enzyme immuno-assay kits (EIA/ELISA kits) for measuring the cytokines/chemokines and mediators may also be used according to instructions by the manufacturer(s). The cell pellets are stored frozen (-80°C) in RLT buffer (QIAGEN, Valencia, CA) until further processing for microarray experiments (see Example 12 above).

In addition to studying the effects of the drugs above on release of mediators and chemokines/cytokines from the neutrophil-like NB4 and HL-60 cells, effects of the drugs on spontaneous or stimulated adhesion and/or migration of these cells may also be analyzed (freshly isolated human blood polymorphonuclear cells (PMN) isolated according to standard protocols may also be used). Spontaneous or stimulated (with fMLP, IL-8, PAF, LTB₄ or other relevant PMN activating factors) adhesion of the PMN or neutrophil-like cells to e.g. cultured endothelial cells or protein-coated artificial surfaces are studied using well established and documented experimental approaches and assays. Migration (stimulated with fMLP, IL-8, PAF, LTB₄ or other relevant PMN chemotactic factors) of the PMN or neutrophil-like cells are studied using well established and documented experimental approaches and assays, e.g. migration through commercially available protein-coated membranes designed for such migration studies.

### Example 18

### Inhibition of MIP-2 Expression by Pemirolast and Ramatroban

Male Sprague-Dawley rats weighing 370-430 g were used. Animals were anesthetized with Isoflurane in oxygen and acute tissue injury and acute inflammation was achieved in the left common carotid artery as follows: After surgical exposure of the left common, external and internal carotid arteries and temporary cessation of blood flow, a balloon catheter (2-French Fogarty) was passed through the external carotid into the aorta. Next, the balloon was inflated with sufficient water to slightly distend the common carotid artery and then pulled back to the external carotid. This procedure was repeated three times, and then the catheter was removed and the wound closed. Test drugs (5 mg/kg pemirolast and 5 mg/kg ramatroban, 5 mg/kg pemirolast alone and 5 mg/kg ramatroban alone) were administered subcutaneously 60 minutes before tissue injury. There were 3 rats in each of these three drug treatment groups. A control treatment group of 3 rats was given saline subcutaneously 60 minutes before tissue injury.

Solutions of mg/ml pemirolast (potassium salt purchased from American Custom Chemicals Corporation, San Diego, USA) and 2.5 mg/ml ramatroban (purchased from Cayman Chemical, Ann Arbor, MI, USA) were prepared in saline, with sonication, warming and, for ramatroban, adjustment of pH (by adding one equivalent of aqueous NaOH) as needed. The final ramatroban solution had a pH of approximately 7.5.

8 hours after injury, the animals were anesthetized with Isoflurane in oxygen and their left carotid arteries exposed. A clamp was put on the very proximal part of the common and internal carotid arteries, respectively, and then the vessel between the clamps was gently flushed with sterile saline, removed and stored frozen at -80°C in TRIzol, and, as described below, subsequently analyzed with regard to tissue gene expression using microarray technology.

Total RNA from the (homogenized) rat tissue was isolated using TRIzol (Invitrogen, Carlsbad, CA) followed by RNeasy cleanup (QIAGEN, Valencia, CA) according to manufacturers' protocols (RNA amplified and labeled according to the two-cycle target labeling protocol). Microarray analysis was performed using GeneChip® Rat Genome 230 2.0 Array (Affymetrix, Santa Clara, CA) according to the manufacturers' protocols. The microarray expression data was analyzed using GeneChip Operating Software (Affymetrix) and Bioconductor/R (www.bioconductor.com). In all tissue samples, the values for the parameters scaling factor, number of present and GAPDH ratio were within the ranges recommended by the manufacturer Affymetrix.

Macrophage inflammatory protein-2 (MIP-2), also designated chemokine (C-X-C motif) ligand 2 (CXCL2), is a potent pro-inflammatory chemokine. MIP-2 gene expression levels in the four different treatment groups were compared by comparing the mean normalized MIP-2 probe-set signal intensity ("MIP-2 signal intensity") of each of the four groups of 3 animals (the signal intensity, which is expressed as a numeric value, is directly related to the expression level of a gene, and the p-value for each individual probe-set signal for MIP-2 was less than 0.01 in all 12 animals). In the saline treated control group, the mean MIP-2 signal intensity was 203. In animals treated with both ramatroban and pemirolast, the corresponding mean MIP-2 signal intensity was 48, i.e. compared to the control group, the combined treatment with ramatroban and pemirolast reduced the MIP-2 gene expression by approximately 75%. For comparison, the corresponding mean MIP-2 signal intensity was 326 in the group treated with ramatroban alone and 710 in the group treated with pemirolast alone, i.e. in contrast to the combination treatment with ramatroban plus pemirolast, ramatroban alone or pemirolast alone did not reduce MIP-2 gene expression levels. Thus, ramatroban and pemirolast in combination synergistically inhibited the vascular MIP-2 expression.

### Example 19

### Inhibition of PDGF expression by Pemirolast and Ramatroban

Equivalent preparative steps to those described in Example 18 were conducted.

Platelet derived growth factor plays a role in e.g. cell proliferation and cell migration and has been linked to several diseases, including atherosclerosis and fibrosis. Platelet derived growth factor, alpha (PDGFa) gene expression levels in the four different treatment groups were compared by comparing the mean normalized PDGFa probe-set signal intensity ("PDGFa signal intensity") of each of the four groups of 3 animals (the signal intensity, which is expressed as a numeric value, is directly related to the expression level of a gene, and the p-value for each individual probe-set signal for PDGFa was less than 0.01 in all 12 animals). In the saline treated control group, the mean PDGFa signal intensity was 528. In animals treated with both ramatroban and pemirolast, the corresponding mean PDGFa signal intensity was 392, i.e. compared to the control group, the combined treatment with ramatroban and pemirolast reduced the PDGFa gene expression by approximately 26%. For comparison, the corresponding mean PDGFa signal intensity was 566 in the group treated with ramatroban alone and 800 in the group treated with pemirolast alone, i.e. in contrast to the combination treatment with ramatroban plus pemirolast, ramatroban alone or pemirolast alone did not reduce PDGFa gene expression levels. Thus, ramatroban and pemirolast in combination synergistically inhibited the vascular PDGFa expression.

### Example 20

### Inhibition of Macrophage Proliferation by Pemirolast and Ramatroban

Cells from the human macrophage cell-line MonoMac-6 (MM6) (Ziegler-Heitbrock et al, Int. J. Cancer, 41, 456 (1988)) were cultured (37°C/5% CO₂) in RPMI-1640 medium supplemented with 1 mM sodium pyruvate, 1×nonessential amino acids, 1-100 µg/mL insulin, 1 mM oxalacetic acid, 100 units/mL penicillin, 100 µg/mL streptomycin and 10% (v/v) fetal bovine serum. At the start of the experiment, MM6 cells were seeded in 96-well plates at a density of 1x10⁵ cells/mL (100 µl per well). Proliferation of the MM6 cells was measured using the Cell Proliferation Reagent WST-1 (Roche Diagnostics Scandinavia AB, Bromma, Sweden) or by cell counting using a microscope. The WST-1 reagent is designed to be used for spectrophotometric quantification of e.g. cell growth in proliferation assays and was used according to the manufacturers' instructions. The wavelength for measuring absorbance was 450 nm and the absorbance of all untreated control cells exposed to the WST-1 reagent was between 0.9 and 2.4. Stock solutions of pemirolast (potassium salt purchased from American Custom Chemicals Corporation, San Diego, USA) and ramatroban (purchased from Cayman Chemical, Ann Arbor, MI, USA as free acid and transferred into sodium salt by adding one equivalent of aqueous NaOH) were made in sterile saline.

Untreated MM6 cells were found to increase from 1x10⁵ cells/mL at the start of the experiment to 1.4x10⁵ ± 0.06x10⁵ cells/mL and 2.3x10⁵ ± 0.10x10⁵ cells/mL after 24 and 48 hours, respectively (mean values ± sem, n=4 for each of the three time-points). The effects of pemirolast, and/or ramatroban (added at the start of the experiments) on MM6 cell proliferation were studied 48 hours after start of the experiments using the WST-1 reagent described above.

Treatment of the MM6 cells with ramatroban at a final concentration of 100 µM resulted in 16.8% inhibition of MM6 proliferation (mean value, n=5) (10 µM ramatroban had an inhibitory effect that was less that that of 100 µM of ramatroban, n=5, data not shown). For comparison, treatment of the MM6 cells with pemirolast at a final concentration of 100 µM resulted in a mean 9.4% inhibition of proliferation (n=5) (10 µM pemirolast had an inhibitory effect very similar to that of 100 µM of pemirolast, n=5, data not shown). When the MM6 cells were treated with a combination of 100 µM pemirolast and 100 µM ramatroban, the MM6 proliferation was synergistically reduced by 39.5% (mean value, n=5). In other words, compared to the 16.8% inhibition caused by ramatroban alone (see above), treatment with 100 µM ramatroban in the presence of 100 µM of pemirolast caused a synergistic 33.2% inhibition of MM6 proliferation compared to treatment with 100 µM pemirolast alone.

One or more of the above-described Examples demonstrate a clear synergistic effect for the combination of pemirolast and ramatroban. Such a combination is thus useful in the treatment of inflammatory disorders, including atherosclerosis and related conditions.

## Claims

1. A combination product comprising:
(a) pemirolast, or a pharmaceutically-acceptable salt or solvate thereof; and
(b) ramatroban, or a pharmaceutically-acceptable salt or solvate thereof.

2. A combination product as claimed in Claim 1, which comprises a pharmaceutical formulation including pemirolast, or a pharmaceutically-acceptable salt or solvate thereof; ramatroban, or a pharmaceutically-acceptable salt or solvate thereof; and a pharmaceutically-acceptable adjuvant, diluent or carrier.

3. A combination product as claimed in Claim 1, which comprises a kit of parts comprising components:
(A) a pharmaceutical formulation including pemirolast, or a pharmaceutically-acceptable salt or solvate thereof, in admixture with a pharmaceutically-acceptable adjuvant, diluent or carrier; and
(B) a pharmaceutical formulation including ramatroban, or a pharmaceutically-acceptable salt or solvate thereof, in admixture with a pharmaceutically-acceptable adjuvant, diluent or carrier,
which components (A) and (B) are each provided in a form that is suitable for administration in conjunction with the other.

4. A method of making a kit of parts as defined in Claim 3, which method comprises bringing component (A) into association with a component (B), thus rendering the two components suitable for administration in conjunction with each other.

5. A kit of parts comprising:
(I) one of components (A) and (B) as defined in Claim 3; together with
(II) instructions to use that component in conjunction with the other of the two components.

6. A kit of parts as claimed in any one of Claim 3 or Claim 5, wherein components (A) and (B) are suitable for sequential, separate and/or simultaneous use in the treatment of an inflammatory disorder.

7. The use of a combination product as defined in any one of Claims 1 to 3, 5 or 6 for the manufacture of a medicament for the treatment of an inflammatory disorder.

8. A combination product as defined in any one of Claims 1 to 3, 5 or 6 for use in the treatment of an inflammatory disorder.

9. The use of components (A) and (B) as defined in Claim 3 or Claim 6 for the manufacture of a medicament for the treatment of an inflammatory disorder, which method comprises administering said components (A) and (B) to a patient in conjunction with each other in combination therapy to treat the inflammatory disorder.

10. Components (A) and (B) as defined in Claim 3 or Claim 6 for use in the treatment of an inflammatory disorder, which method comprises administering said components (A) and (B) to a patient in conjunction with each other in combination therapy to treat the inflammatory disorder.

11. A kit of parts as claimed in Claim 6, a use as claimed in Claim 7 or Claim 9, a combination product as claimed in Claim 8, or components as claimed in Claim 10, wherein the disorder is selected from migraine, asthma, chronic obstructive pulmonary disease, Crohn's disease, multiple sclerosis, psoriasis, rheumatoid arthritis, systemic lupus erythematosus or ulcerative colitis.

12. A kit of parts as claimed in Claim 6, a use as claimed in Claim 7 or Claim 9, a combination product as claimed in Claim 8, or components as claimed in Claim 10, wherein the disorder is atherosclerosis or an associated cardiovascular disorder.

13. A kit of parts, a use, a combination product or components as claimed in Claim 12, wherein the disorder is atherosclerosis.

14. A kit of parts, a use, a combination product or components as claimed in Claim 12, wherein the cardiovascular disorder associated with atherosclerosis is selected from an aortic aneurysm, arteriosclerosis, peripheral arterial occlusive disease, a coronary artery disease, a coronary disease, plaque rupture, atheroma rupture and/or instability, a vascular disease, an arterial disease, an ischemic disease, ischemia and stroke.

15. A kit of parts a use, a combination product or components as claimed in Claim 14, wherein the cardiovascular disorder is an aortic aneurysm.

16. A kit of parts, a use, a combination product or components as claimed in Claim 14 wherein:
the coronary artery disease is selected from angina pectoris, myocardial infarction and heart attack;
the coronary disease is selected from a cardiac disease and a heart disease; and/or
the stroke is selected from cerebro-vascular accident and transient ischaemic attack.

17. A kit of parts, a use, a combination product or components as claimed in and one of Claims 6 to 16, wherein the patient has an acute coronary syndrome.

## Patentansprüche

1. Kombinationsprodukt, welches umfasst:
a) Pemirolast oder ein pharmazeutisch akzeptables Salz oder Solvat davon; und
b) Ramatroban oder ein pharmazeutisch akzeptables Salz oder Solvat davon.

2. Kombinationsprodukt nach Anspruch 1, welches eine pharmazeutische Zusammensetzung aufweist, die Pemirolast oder ein pharmazeutisch akzeptables Salz oder Solvat davon, Ramatroban oder ein pharmazeutisch akzeptables Salz oder Solvat davon und ein pharmazeutisch akzeptables Adjuvans, Verdünnungsmittel oder Trägerstoff einschließt.

3. Kombinationsprodukt nach Anspruch 1, welches ein Kit von Teilen umfasst, die Komponenten enthaltenden:
(A) eine pharmazeutische Zusammensetzung einschließlich Pemirolast oder ein pharmazeutisch akzeptables Salz oder Solvat davon in Beimischung mit einem pharmazeutisch akzeptablen Adjuvans, Verdünnungsmittel oder Trägerstoff; und
(B) eine pharmazeutische Zusammensetzung, die Ramatroban oder ein pharmazeutisch akzeptables Salz oder Solvat davon in Beimischung mit einem pharmazeutisch akzeptablen Adjuvans, Verdünnungsmittel oder Trägerstoff einschließt,
dessen Komponenten (A) und (B) jeweils in einer Form vorgesehen sind, die geeignet ist für die Verabreichung in Verbindung mit der anderen.

4. Verfahren zur Herstellung eines Kits aus Teilen wie in Anspruch 3 definiert, wobei das Verfahren das in-Verbindung-Bringen einer Komponente (A) mit einer Komponente (B) umfasst, wodurch die zwei Komponenten für die Verabreichung in Verbindung miteinander gemacht werden.

5. Kit aus Teilen umfassend:
(I) eine der Komponenten (A) und (B) wie in Anspruch 3 definiert; zusammen mit
(II) Anweisungen diese Komponente in Verbindung mit der anderen der beiden Komponenten zu verwenden.

6. Kit aus Teilen wie in einem der Ansprüche 3 oder 5 beansprucht, bei dem Komponenten (A) und (B) für sequenzielle, separate und/oder simultane Verwendung bei der Behandlung einer entzündlichen Erkrankung geeignet sind.

7. Verwendung eines Kombinationsprodukts wie in einem der Ansprüche 1 bis 3, 5 oder 6 definiert, für die Herstellung eines Medikaments für die Behandlung einer entzündlichen Erkrankung.

8. Kombinationsprodukt wie in einem der Ansprüche 1 bis 3, 5 oder 6 definiert, zur Verwendung bei der Behandlung einer entzündlichen Erkrankung.

9. Verwendung von Komponenten (A) und (B) wie in Anspruch 3 oder Anspruch 6 definiert, für die Herstellung eines Medikamentes zur Behandlung einer entzündlichen Erkrankung, welches Verfahren die Verabreichung genannter Komponenten (A) und (B) an einen Patienten in Verbindung miteinander als Kombinationstherapie zum Behandeln der entzündlichen Erkrankung umfasst.

10. Komponenten (A) und (B) wie in Anspruch 3 oder Anspruch 6 definiert, zur Verwendung bei der Behandlung einer entzündlichen Erkrankung, welches Verfahren die Verabreichung genannter Komponenten (A) und (B) an einen Patienten in Verbindung miteinander als Kombinationstherapie zum Behandeln der entzündlichen Erkrankung umfasst.

11. Kit aus Teilen wie in Anspruch 6 beansprucht, Verwendung wie in Anspruch 7 oder Anspruch 9 beansprucht, Kombinationsprodukt wie in Anspruch 8 beansprucht, oder Komponenten wie in Anspruch 10 beansprucht, wobei die Erkrankung ausgewählt ist aus Migräne, Asthma, chronische obstruktive Lungenentzündung, Morbus Crohn, Multiple Sklerose, Schuppenflechte, rheumatische Arthritis, systemischer Lupus Erythematosus oder Colitis Ulcerosa.

12. Kit aus Teilen wie in Anspruch 6 beansprucht, Verwendung wie in Anspruch 7 oder Anspruch 9 beansprucht, Kombinationsprodukt wie in Anspruch 8 beansprucht, oder Komponenten wie in Anspruch 10 beansprucht, wobei die Erkrankung Atherosklerose oder eine damit verbundene cardiovasculäre Erkrankung ist.

13. Kit aus Teilen, Verwendung, Kombinationsprodukt oder Komponenten wie in Anspruch 12 beansprucht, wobei die Erkrankung Atherosklerose ist.

14. Kit aus Teilen, Verwendung, Kombinationsprodukt oder Komponenten wie in Anspruch 12 beansprucht, wobei die cardiovasculäre Erkrankung in Verbindung mit Atherosklerose ausgewählt ist aus Aortenaneurysma, Atherosklerose, periphere arterielle Verschlusskrankheit, Koronararterienerkrankung; koronare Krankheit, Plaque-Ruptur, Atherom-Ruptur und/oder -Instabilität, Gefäßerkrankung, Arterienerkrankung, ischämische Erkrankung, Ischämie und Schlaganfall.

15. Kit aus Teilen, Verwendung, Kombinationsprodukt oder Komponenten wie in Anspruch 14 beansprucht, wobei die cardiovasculäre Erkrankung ein Aortenaneurysma ist.

16. Kit von Teilen, Verwendung, Kombinationprodukt oder Komponenten wie in Anspruch 14 beansprucht, wobei:
die koronare Arterienerkrankung ausgewählt ist aus Angina Pectoris, Myocardial-Infarkt und Herzinfarkt;
die Koronarerkrankung ausgewählt ist aus Herzerkrankung und Herzleiden; und/oder
der Schlaganfall ausgewählt ist aus Cerebro-Vasculäre Störung und transitorische ischämische Attacke.

17. Kit aus Teilen, Verwendung, Kombinationsprodukt oder Komponenten wie in einem der Ansprüche 6 bis 16 beansprucht, wobei der Patient ein akutes Koronar-Syndrom hat.

## Revendications

1. Produit de combinaison comprenant :
(a) du pémirolast, ou un sel ou solvate pharmaceutiquement acceptable de celui-ci ; et
(b) du ramatroban, ou un sel ou solvate pharmaceutiquement acceptable de celui-ci.

2. Produit de combinaison selon la revendication 1, qui comprend une formulation pharmaceutique incluant du pémirolast, ou un sel ou solvate pharmaceutiquement acceptable de celui-ci ; du ramatroban, ou un sel ou solvate pharmaceutiquement acceptable de celui-ci ; et un adjuvant, diluant ou véhicule pharmaceutiquement acceptables.

3. Produit de combinaison selon la revendication 1, comprenant un ensemble de pièces comprenant les composants :
(A) une formulation pharmaceutique incluant du pémirolast, ou un sel ou solvate pharmaceutiquement acceptable de celui-ci, en mélange avec un adjuvant, diluant ou véhicule pharmaceutiquement acceptables ; et
(B) une formulation pharmaceutique incluant du ramatroban, ou un sel ou solvate pharmaceutiquement acceptable de celui-ci, en mélange avec un adjuvant, diluant ou véhicule pharmaceutiquement acceptables,
lesquels composants (A) et (B) sont chacun fournis sous une forme qui est appropriée pour une administration conjointe l'un avec l'autre.

4. Procédé de fabrication d'un ensemble de pièces tel que défini dans la revendication 3, lequel procédé comprenant le fait d'associer le composant (A) au composant (B), rendant ainsi les deux composants appropriés pour une administration conjointe l'un avec l'autre.

5. Ensemble de pièces comprenant :
(I) un des composants (A) et (B) tels que définis dans la revendication 3 ; conjointement avec
(II) des instructions pour l'utilisation conjointe de ce composant avec l'autre des deux composants.

6. Ensemble de pièces selon l'une quelconque de la revendication 3 ou de la revendication 5, dans lequel les composants (A) et (B) sont appropriés pour une utilisation séquentielle, séparée et/ou simultanée dans le traitement d'un trouble inflammatoire.

7. Utilisation d'un produit de combinaison tel que défini dans l'une quelconque des revendications 1 à 3, 5 ou 6, pour la fabrication d'un médicament destiné au traitement d'un trouble inflammatoire.

8. Produit de combinaison selon l'une quelconque des revendications 1 à 3, 5 ou 6 pour une utilisation dans le traitement d'un trouble inflammatoire.

9. Utilisation des composants (A) et (B) tels que définis dans la revendication 3 ou la revendication 6 pour la fabrication d'un médicament destiné au traitement d'un trouble inflammatoire, lequel traitement comprend l'administration conjointe desdits composants (A) et (B) à un patient en polythérapie pour traiter le trouble inflammatoire.

10. Composants (A) et (B) tels que définis dans la revendication 3 ou la revendication 6, pour une utilisation dans le traitement d'un trouble inflammatoire, lequel traitement comprend l'administration conjointe desdits composants (A) et (B) à un patient en polythérapie pour traiter le trouble inflammatoire.

11. Ensemble de pièces selon la revendication 6, utilisation selon la revendication 7 ou la revendication 9, produit de combinaison selon la revendication 8, ou composants selon la revendication 10, dans lesquels le trouble est choisi parmi une migraine, l'asthme, une broncho-pneumopathie chronique obstructive, la maladie de Crohn, la sclérose en plaques, le psoriasis, la polyarthrite rhumatoïde, le lupus érythémateux systémique ou la rectocolite hémorragique.

12. Ensemble de pièces selon la revendication 6, utilisation selon la revendication 7 ou la revendication 9, produit combiné selon la revendication 8, ou composants selon la revendication 10, dans lesquels le trouble est l'athérosclérose ou un trouble cardiovasculaire associé.

13. Ensemble de pièces, utilisation, produit combiné ou composants selon la revendication 12, dans lesquels le trouble est l'athérosclérose.

14. Ensemble de pièces, utilisation, produit combiné ou composants selon la revendication 12, dans lesquels le trouble cardiovasculaire associé à l'athérosclérose est choisi parmi un anévrisme aortique, l'artériosclérose, une maladie occlusive artérielle périphérique, une insuffisance coronarienne, une maladie coronarienne, une rupture de plaque, une rupture et/ou une instabilité d'athérome, une maladie vasculaire, une maladie artérielle, une maladie ischémique, une ischémie et une attaque.

15. Ensemble de pièces, utilisation, produit combiné ou composants selon la revendication 14, dans lesquels le trouble cardiovasculaire est un anévrisme aortique.

16. Ensemble de pièces, utilisation, produit combiné ou composants selon la revendication 14, dans lesquels :
l'insuffisance coronarienne est choisie parmi une angine de poitrine, un infarctus du myocarde et une attaque cardiaque ;
la maladie coronarienne est choisie parmi une maladie cardiaque et une maladie du coeur; et/ou
l'attaque est choisie parmi un accident vasculaire cérébral et une attaque ischémique transitoire.

17. Ensemble de pièces, utilisation, produit combiné ou composants selon l'une des revendications 6 à 16, dans lesquels le patient est atteint d'un syndrome coronarien aigu.
